# EUROPEAN PATENT APPLICATION

(11) **EP 4 029 484 A1**
(43) Date of publication of application: **20.07.2022**
(21) Application number: 21199465.2
(22) Date of filing: 28.09.2021
(51) Int. Cl.: A61F 13/15, A61F 13/539

(54) **A METHOD AND AN APPARATUS FOR BINDING TOGETHER A PLURALITY OF ELEMENTS OF AN ABSORBENT SANITARY ARTICLE**

(30) Priority: 15.01.2021 IT 202000025696
(71) Applicant: GDM S.P.A., 40133 Bologna (IT)
(72) Inventor: PIANTONI, Matteo, 24021 Albino (BG) (IT); ZAVALLONI, Alessandro, 26028 Sesto ed Uniti (CR) (IT); ROSANI, Marco, 26019 Vailate (CR) (IT); GIARDINI, Francesco, 24058 Romano di Lombardia (BG) (IT)
(74) Representative: Porta & Consulenti Associati S.p.A.

(57) **Abstract**

A method for binding together a plurality of elements of absorbent sanitary articles, comprising feeding first primary elements (100) in the form of a first continuous web (A1) onto a forming support (10), feeding a sequence of intermediate elements (101a, 101b) in the form of discrete elements onto the forming support (10) in an at least partial overlapping relation with respect to the first primary elements (100), holding the intermediate elements (101a, 101b) onto the forming support (10), preventing a relative sliding between the intermediate elements (101a, 101b) and the first primary elements (100), feeding the second primary elements (102) in the form of a second continuous web onto the forming support (10) in an at least partial overlapping relation with respect to the first primary elements (100) and the intermediate elements (101a, 101b) and simultaneously binding together the first primary elements (100), the intermediate elements (101a, 101b) and the second primary elements (102) along at least one mutual overlapping region thereof arranged onto the forming support (10).

## Description

The present invention relates to a method and an apparatus for binding together a plurality of elements of an absorbent sanitary article.

Absorbent sanitary articles, such as baby nappies, diapers and napkins for incontinent persons and the like typically have a front part, which can be worn on the front of the wearer's body, a back part, which can be worn on the back of the wearer's body, and a middle part arranged between the front and the back and wearable between the wearer's legs.

Structurally, an absorbent sanitary article comprises at least two primary elements extending longitudinally from the front to the back of the absorbent sanitary article. The primary elements include an upper and a lower element, respectively called "topsheet" and "backsheet" in industry jargon, overlapping and bound together. Both the upper and lower element are essentially rectangular in shape.

The upper element is overlapped on the lower element and is configured to be arranged in a contiguous position to the wearer's body when the absorbent sanitary article is worn by the latter. The upper element comprises a central portion which, in use, comes into contact with the user's skin, or close thereto, so as to form a hollow space between the absorbent sanitary article and the user's body. The central portion is configured to receive bodily excrement and is made of a material permeable to body fluids. The upper element can also comprise two side strips, also called "standing gather", configured to ergonomically encircle the user's legs to reduce the clearance between the absorbent sanitary article and the user's body. The side strips can be made of a soft material suitable for contact with the user's skin, for example a non-woven fabric. The upper element can also comprise elastic elements coupled to the side strips so as to further reduce the aforementioned clearance, thus reducing the risk of excrement escaping from the aforementioned gap.

The lower element comprises a layer impermeable to body fluids, e.g., made of polyethylene or bioplastic. The lower element can incorporate an additional reinforcing layer associated with the waterproof layer and configured to improve the tear resistance of the absorbent article. The lower element can also incorporate one or more aesthetic components configured to externally cover the absorbent sanitary article when worn.

An absorbent core is arranged between the upper and lower elements. Such an absorbent core has the function of absorbing body fluids passing through the upper element and holding them without leaks, even for several hours. The absorbent core comprises one or more super-absorbent polymers (SAP), e.g., sodium polyacrylate, normally in granular form, arranged within a cellulose or other similar fibre covering.

Intermediate elements can be interposed between the upper and lower elements. For example, a capture and distribution element can be arranged between the upper element and the absorbent core. The capture and distribution element is configured to receive body fluids passing through the upper element and distribute them over the absorbent core quickly and evenly, so as to move such fluids away from the user's skin and improve comfort. For this purpose, the capture and distribution element is made of a material permeable to body fluids and configured to rapidly capture and distribute such fluids. For example, the capture and distribution element can be made of a non-woven fabric comprising in whole or in part cellulose fibres, polyester fibres or the like. In fact, the capture and distribution element determines a physical separation between the upper element and the absorbent core, preventing the fluids contained in the absorbent core from wetting the upper element. The capture and distribution element can be bound to the upper element, in which case, for the purposes of the present patent application, it is considered an integral part of the upper element.

Additional intermediate elements, such as flaps (or side panels), are arranged between the upper and lower elements. Such flaps are bound to both the upper and lower elements so that they project laterally from such elements. Typically there is a pair of front flaps bound to the front of the absorbent sanitary article and a pair of rear flaps bound to the back of the absorbent sanitary article. Each front flap is configured to be reversibly coupled, e.g., by means of adhesives or Velcro or the like, to a respective rear flap, so as to encircle the user's pelvis or waist and firmly and comfortably fix the absorbent sanitary article to the user's body. The flaps can be made of elastic, tensile-resistant materials.

Typically, the absorbent articles of the type described above are made in a production line from continuous webs, from which the upper, middle and lower elements are cut.

EP 3 300 708 A1 describes an absorbent sanitary article in which the side panels are bound at regular intervals onto opposite sides of a topsheet advancing in a machine direction. Prior to the application of the topsheet to the backsheet, first welds are made in order to join the side panels to the topsheet, so that they remain integral with the topsheet while the latter advances in the machine direction. Next, the topsheet is overlapped on the backsheet and a second weld is made to join the topsheet and backsheet together.

The Applicant has observed that, during the use of the absorbent sanitary articles, certain intermediate elements, such as the flaps, are subjected to tensile forces by the user.

The Applicant has noted that in an absorbent sanitary article of the type described in EP 3 300 708 A1, such tensile forces generate shear forces acting mainly on the topsheet, to which the intermediate elements are directly bound.

The Applicant considers that the aforesaid shear forces could lead to a separation of the topsheet from the backsheet at the areas where such elements join the intermediate elements, with consequent damage to the absorbent sanitary article.

The Applicant has perceived that such damage could be avoided if the aforesaid shear forces were equally distributed on the topsheet and backsheet.

The Applicant has found that by binding the two primary elements and the intermediate elements together at the same time, i.e., in a single operating step, the intermediate elements are associated with the two primary elements in essentially the same manner. In this case, the aforesaid shear forces are transferred evenly and equally to both primary elements, increasing the reliability of the product.

Therefore, the present invention relates, in a first aspect, to a method for binding together a plurality of elements of absorbent sanitary articles.

Preferably, it is included to feed primary elements of absorbent sanitary articles onto a forming support.

Preferably, said primary elements are in the form of a first continuous web.

Preferably, it is included to feed a sequence of intermediate elements of absorbent sanitary articles onto the forming support in an at least partial overlapping relation with respect to the first primary elements.

Preferably, said intermediate elements are in the form of discrete elements.

Preferably, it is included to hold said intermediate elements onto the forming support, preventing a relative sliding between said intermediate elements and said primary elements.

Preferably, it is included to feed second primary elements of absorbent sanitary articles onto the forming support in an at least partial overlapping relation with respect to the first primary elements and intermediate elements.

Preferably, said second primary elements are in the form of a second continuous web.

Preferably, it is included to simultaneously hold together the first primary elements, the intermediate elements and the second primary elements along at least one of the overlapping regions thereof arranged on the forming support.

In the following of the present description and in the subsequent claims, "continuous web" means a flexible long-form element having a longitudinal dimension (length) which is at least one order of magnitude larger than a first transverse dimension (width) perpendicular to the longitudinal direction and at least two orders of magnitude larger than a second transverse dimension (thickness) perpendicular to the first transverse dimension and the longitudinal direction. Such a flexible element extends longitudinally without interruption for a length which can form a plurality of respective primary elements, such as topsheet and backsheet, in succession. Such a flexible element can be combined with other continuous or discrete elements, such as side strips, elastic elements, capture and distribution layers or absorbent cores.

"Discrete elements" means elements which are separate from each other and have longitudinal dimensions less than, or at most of the same order of magnitude as, those of a single absorbent sanitary article.

"Mutual overlapping region" means a region in which a portion of the second primary elements is overlapped on a portion of the discrete elements which in turn is overlapped on a portion of the first primary elements.

The first primary elements are preferably topsheets of absorbent sanitary articles.

The second primary elements are preferably backsheets of absorbent sanitary articles.

The intermediate elements are preferably the front and/or rear flaps of absorbent sanitary articles. Alternatively, the intermediate elements can comprise further components of absorbent sanitary articles, such as capture and distribution layers or reinforcing layers or other discrete elements typically included in absorbent sanitary articles between the topsheet and backsheet.

The Applicant has found that by holding the first primary elements and the intermediate elements onto the forming support, preventing a relative sliding between the intermediate elements and the first primary elements, it is possible to transport the intermediate elements integral with the first primary elements to the position where the second primary elements are fed thereon.

The Applicant has found that it is thereby possible to simultaneously bind together the first primary elements, the intermediate elements and the second primary elements at least along a mutual overlapping region, ensuring that the intermediate elements are joined in a substantially identical manner to the first primary elements and the second primary elements.

In a second aspect, the present invention relates to an apparatus for binding together a plurality of elements of absorbent sanitary articles.

Preferably, a forming support movable along a working direction is included.

Preferably, a first feeding member configured to feed primary elements of absorbent sanitary articles onto the forming support at a first deposition position is included.

Preferably, said primary elements are in the form of a first continuous web.

Preferably, at least a second feeding member configured to feed a sequence of intermediate elements of absorbent sanitary articles onto the forming support is included.

Preferably, such feeding occurs at a deposition area arranged downstream of said first deposition position with reference to said working direction.

Preferably, said intermediate elements are in the form of discrete elements.

Preferably, a third feeding member configured to feed second primary elements of absorbent sanitary articles onto the forming support is included.

Preferably, such feeding occurs at a second deposition position located downstream of said deposition area with reference to said working direction.

Preferably, said second primary elements are in the form of a second continuous web.

Preferably, a bonding device active onto the forming support is included at or downstream of said second deposition position with reference to said working direction of the forming support.

Preferably, said bonding device is configured to bind together the first primary elements, the sequence of intermediate elements and the second primary elements along at least one of the mutual overlapping regions thereof defined onto the forming support.

In the following description and in the subsequent claims, the expressions "downstream" and "upstream" refer to the working direction, which corresponds to the direction of movement of the forming support. Therefore, assuming for example a straight working direction from left to right, a "downstream" position with respect to an element or reference point is any position to the right of said element or reference point and an "upstream" position with respect to an element or reference point is any position to the left of said element or reference point. In the case where the working direction is circular, referring for example to a clockwise rotation, a "downstream" position with respect to an element or reference point is any position following such an element or reference point and an "upstream" position with respect to an element or reference point is any position preceding said element or reference point.

The present invention may have, in both aspects discussed above, at least one of the preferred features described below. Such features may therefore be present singly or in combination, except where expressly stated otherwise, either in the method of the first aspect of the present invention or in the apparatus of the second aspect of the present invention.

Preferably, before or after feeding the first primary elements onto the forming support, it is included to move the forming support along a working direction.

Preferably, after feeding the first primary elements onto the forming support, it is included to hold said primary elements onto the forming support. For this purpose, special holding members are included at the forming support.

Preferably, such a holding is suitable for preventing a relative sliding between the primary elements and the forming support during the movement of the forming support along said working direction. Thereby, the primary elements adhere to the forming support and maintain the position thereof with respect to the forming support during the movement of the latter along the working direction, thus also being moved synchronously to the forming support.

Preferably, holding said primary elements onto the forming support comprises exerting a suction action on said primary elements towards the forming support.

The suction action enables holding the first primary elements onto the forming support temporarily, i.e., until such time as the first primary elements are bound to the intermediate elements and the second primary elements and are removed together with the intermediate elements and the second primary elements from the forming support.

Preferably, exerting a suction action on said first primary elements comprises operating an active suction device on said first primary elements through a plurality of holding holes formed on a deposition surface of the forming support.

The holding holes create a vacuum near the deposition surface, attracting the first primary elements onto the forming support.

Preferably, said vacuum is between 1,000 and 12,000 Pa, even more preferably between 2,000 and 10,000 Pa.

Preferably, the aforesaid holding members are configured to hold in position onto the forming support both the primary and intermediate elements during the movement of the forming support along said working direction.

Preferably, holding said intermediate elements onto the forming support comprises exerting said suction action on said intermediate elements.

Thereby, the same suction action enables both the primary and intermediate elements to be held onto the forming support.

Preferably, the aforesaid holding members are configured to hold the second primary elements in place onto the forming support as well.

Preferably, the second primary elements are held onto the forming support while the first primary elements, intermediate elements and second primary elements are bound together.

Such a hold helps keep the aforesaid elements well adhered together in the mutual overlapping region, allowing a reciprocal bond between the aforesaid elements to be achieved in a single operating step and through a single bonding device.

Preferably, the primary elements comprise at least one central portion and two side strips bound at opposite sides of the central portion.

Preferably, the first primary elements, intermediate elements and second primary elements are bound together, and the side strips, intermediate elements and second primary elements are bound together by the aforesaid bonding device.

Preferably, the bonding device is arranged below the forming support.

Preferably, the bonding device is arranged at a plane of vertical symmetry of the forming support.

Preferably, the forming support comprises a drum rotatable around a rotation axis.

In such a case, preferably, imagining a clock face overlapped on the drum, the bonding device is preferably arranged in a position corresponding to 6 o'clock.

Thereby, after the first primary elements, intermediate elements and second primary elements have been bound together, they can be picked up from the forming support and fed to a horizontal conveyor belt.

In an alternative embodiment, the forming support comprises a conveyor belt.

Preferably, after the first primary elements, intermediate elements and second primary elements have been bound together, it is included to further hold the first primary elements, intermediate elements and second primary elements bound together onto the forming support before being removed from the forming support.

This prevents changes in direction immediately after the first primary elements, intermediate elements and second primary elements have been bound together. Such changes in direction could lead to relative movements between the first primary elements, intermediate elements and second primary elements, to the detriment of the quality of the final product.

Preferably, further holding the first primary elements, intermediate elements and second primary elements bound together onto the forming support comprises exerting on said first primary elements, intermediate elements and second primary elements bound together said suction action towards the forming support.

With the same suction action it is therefore possible to hold the various elements of the absorbent sanitary article onto the forming support both before such elements are bound together and after they are bound together.

Preferably, the suction action is exerted continuously onto the forming support between a holding start position and a holding end position.

Preferably, the holding start position is arranged at or upstream of the first deposition position, i.e., the position in which the first primary elements are deposited onto the forming support.

Preferably, the holding end position is arranged at or downstream of the bonding device along the working direction.

Preferably, in the case where the forming support is a drum, the suction device is active along the working direction on a cylindrical surface of the drum, which defines the aforesaid deposition surface, for a circumferential extension of less than 360°.

Therefore, the suction area does not affect the entire cylindrical surface of the drum, but there is a portion of the cylindrical surface which, not being affected by the suction action during the rotation of the drum, allows the separation of the first primary elements, the intermediate elements and the second primary elements from the forming support after they have been mutually bound.

Preferably, the first primary elements are at least partially permeable to air at least in the mutual overlapping region with the intermediate elements and the second primary elements. In particular, the air acting on the first primary elements partly attracts the first primary elements towards the forming support and partly passes through them to act on the intermediate elements, in the mutual overlapping region, and on the second primary elements, in the area where the second primary elements are overlapped on the first primary elements without the interposition of the intermediate elements.

Thereby, the air suctioned towards the forming support can either hold the first primary elements onto the forming support or pass through the first primary elements and compact the intermediate elements on top of the first primary elements, achieving the desired holding in place of the intermediate elements on the first primary elements.

Preferably, the intermediate elements are at least partly permeable to air at the respective portions over which a second primary element overlaps. In particular, the air acting on the intermediate elements partly draws the intermediate elements towards the forming support and partly passes through them to act on the second primary elements in the mutual overlapping region.

Thereby, the air suctioned towards the forming support can either hold the intermediate elements in position with respect to the first primary elements and the forming support, or pass through the intermediate elements and compact the second primary elements onto the intermediate elements and the first primary elements, achieving the desired holding in position of the second primary elements onto the intermediate elements and the first primary elements, and therefore onto the forming support.

Preferably, the second primary elements are essentially impermeable to air.

The impermeability to air of the second primary elements allows the suction action to have a particularly accentuated holding and compacting effect of the second primary elements on the underlying elements, improving the reciprocal adherence of all the elements at both the mutual overlapping regions and the regions in which the second primary elements are overlapped onto the first primary elements without the interposition of the intermediate elements, thus reducing the possibility of reciprocal movement of the aforesaid elements before they are reciprocally bound.

Preferably, the primary elements are made of a fabric having, at least at the side strips, a grammage between 10 gsm and 20 gsm (grams per square metre), more preferably between 13 gsm and 17 gsm, e.g., 15 gsm.

Preferably, the intermediate elements are made of a fabric having, at least at the portion thereof intended to overlap the primary elements, a greater weight than that of the primary elements.

Preferably, the second primary elements are made of a fabric having, at least at the portion thereof intended to overlap the first primary elements and intermediate elements, a weight greater than that of the first primary elements, e.g., 20 gsm.

The Applicant has found that the above grammage values effectively achieve the holding in position and compacting of the first primary elements, intermediate elements and second primary elements onto the forming support.

Preferably, feeding the sequence of intermediate elements comprises depositing said intermediate elements onto the forming support at least a deposition area arranged downstream of said first deposition position with reference to said working direction.

Preferably, depositing said intermediate elements onto the forming support comprises depositing a first pair of intermediate elements at a first position of said deposition area.

Preferably, depositing said intermediate elements onto the forming support comprises depositing a second pair of intermediate elements at a second position of said deposition area arranged downstream of said first position of said deposition area of discrete elements.

Thereby, intermediate elements of different shapes or types can be deposited at least partial overlapping the primary elements.

Preferably, the first pair of intermediate elements comprises front flaps of an absorbent sanitary article. In such a case, the first pair of intermediate elements is preferably made of a fabric having a grammage between 30 gsm and 80 gsm, even more preferably between 40 gsm and 60 gsm, e.g., 50 gsm.

Preferably, the second pair of intermediate elements comprises rear flaps of an absorbent sanitary article. In such a case, the second pair of intermediate elements is preferably made of a fabric having a grammage between 50 gsm and 130 gsm, even more preferably between 70 gsm and 100 gsm, e.g., 70 gsm or 80 gsm or 90 gsm.

Preferably, feeding the sequence of intermediate elements comprises feeding intermediate elements in the form of at least a third continuous web to at least one deposition member arranged near the forming support.

Preferably, it is included to cut said at least a third continuous web on the at least one deposition member to create intermediate elements in the form of discrete elements and transfer said intermediate elements from the at least one deposition member to the forming support.

Preferably, such a transfer comprises exerting a suction action on the intermediate elements.

Preferably, said at least a second feeding member comprises a first deposition member configured to deposit said first pair of intermediate elements onto the forming support at said first position of said deposition area.

Preferably, said at least a second feeding member comprises a second deposition member configured to deposit said second pair of intermediate elements at said second position of said deposition area, said second position being arranged downstream of said first position of said deposition area with reference to said working direction.

Preferably, said at least a second feeding member comprises a transfer drum adjacent to the forming support and configured to receive intermediate elements in the form of a third continuous belt and deposit intermediate elements in the form of discrete elements onto the forming support.

Preferably, said at least a second feeding member comprises a cutting member active on said transfer drum and configured to separate the intermediate elements from the third continuous web.

Preferably, feeding the second primary elements comprises arranging the second primary elements in a position corresponding to that in which the first primary elements, the intermediate elements and the second primary elements are bound together, and thus between the forming support and the bonding device.

In other words, the first continuous web and the sequence of intermediate elements are preferably fed onto the forming support and held in the respective reciprocal position thereof in which they will be bound together. The second continuous web is preferably fed so as to overlap the first continuous web and the intermediate elements at the operating position in which the elements are then bound together, without a preliminary positioning onto the forming support. Thereby, the second primary elements are bound to the first primary elements and intermediate elements at the same time as, or immediately after, the overlapping thereof, and there is no risk of defects caused by unwanted displacements of the second primary elements with respect to the first primary elements and intermediate elements after they have been fed onto the forming support.

Preferably, the second primary elements are fed along a trajectory which, at the aforesaid operating position, is substantially tangent to the forming support, more preferably substantially horizontal.

Thereby, the second continuous web undergoes essentially zero deflection while being fed to the forming support. As a result, the positioning thereof is more accurate and the possibility of problems such as the accidental formation of folds is reduced.

In an alternative embodiment, the second primary elements are fed onto the forming support at a deposition position which, with reference to the working direction, is arranged upstream of an operating position corresponding to that in which the first primary elements, the intermediate elements and the second primary elements are bound together.

In such a case, the second primary elements are preferably held onto the forming support, preventing a relative sliding between said second primary elements and said first primary elements, until the first primary elements, the intermediate elements and the second primary elements are bound together.

Preferably, the first feeding member comprises a transfer drum. Alternatively, the first feeding member comprises a conveyor belt, a tensioning roller or the like.

Preferably, the third feeding member comprises a conveyor belt. Alternatively, the third feeding member comprises a transfer drum, a tensioning roller or the like.

Preferably, binding together the first primary elements, intermediate elements and second primary elements comprises welding the first primary elements, intermediate elements and second primary elements together.

The bonding device therefore preferably comprises a welding device.

The welding operation can be carried out in a single operating step and allows a stable and permanent reciprocal union between the first primary elements, the intermediate elements and the second primary elements.

Preferably, welding the first primary elements, the intermediate elements and the second primary elements together comprises pressing a welding device against the forming support at the at least one mutual overlapping region while feeding the first primary elements, the intermediate elements and the second primary elements between the welding device and the forming support.

Thereby, the welding device can act through the second primary elements on the elements below and bind the first primary elements, the intermediate elements and the second elements together in a single operating step, maintaining the desired reciprocal positioning.

Preferably, welding together the first primary elements, the intermediate elements and the second primary elements comprises welding the aforesaid elements along a main extension direction of the first continuous web and the second continuous web.

The continuous welding thus binds the continuous webs along the entire length thereof.

Preferably, the aforesaid welding comprises first welding sections passing through the first primary elements, intermediate elements and second primary elements and second welding sections adjacent to the first sections and passing only through the first primary elements and second primary elements.

In other words, in the aforesaid mutual overlapping regions, the welding passes through the second primary elements, the intermediate elements and the first primary elements, while in the regions in which the first and the second continuous web are overlapped without the interposition of intermediate elements, the welding passes only through the second primary elements and the first primary elements.

Preferably, it is included to weld along the opposite side portions of the first primary elements and second primary elements.

Thereby, two substantially parallel welds are obtained which, in addition to binding the first primary elements, the intermediate elements and the second primary elements together, laterally close a central portion of the absorbent article defined between the first primary elements and the second primary elements and destined to house further components of the absorbent sanitary article, such as an absorbent core previously associated with the second continuous web.

Preferably, the welding is an ultrasonic welding. Such a type of welding allows the materials of the first primary elements, intermediate elements and second primary elements to be fused together in a precise and localised manner.

Preferably, the welding device comprises a sonotrode which is vibrated at a frequency between 15 kHz and 25 kHz.

The first primary elements, the intermediate elements and the second primary elements, once bound together, define a continuous web assembly which is picked up from the forming support to be subjected to subsequent operations, including a repeated cutting operation at regular and predetermined time intervals to obtain a plurality of absorbent sanitary articles.

Preferably, a pick-up device is included to pick up said continuous web assembly from the forming support.

Preferably, the pick-up device is configured to separate the aforesaid continuous web assembly from the forming support substantially at the holding end position.

Preferably, it is included that said continuous web assembly is moved away from the forming support along a substantially horizontal trajectory.

After being picked up from the forming support, the continuous web assembly is then fed in a horizontal direction so as to promote the support thereof towards the subsequent steps of the production process of the absorbent sanitary article.

Further characteristics and advantages of the present invention will become clearer from the following detailed description of a preferred embodiment thereof, with reference to the appended drawings and provided by way of indicative and non-limiting example, in which:
- figure 1 is a schematic side view of an apparatus for binding together a plurality of elements of absorbent sanitary articles in accordance with the present invention;
- figure 2 is a schematic side view of an embodiment alternative to that of figure 1;

- figure 3 is a schematic plan view of a first portion of the apparatus of figure 1 or 2 in an operating condition thereof;
- figure 4 is a schematic plan view of a second portion of the apparatus of figure 1 or 2 in an operating condition thereof.

In the appended drawings, an apparatus for binding together a plurality of elements of absorbent sanitary articles in accordance with the present invention is generically indicated with the numerical reference 1 and hereinafter referred to by the abbreviated notation "apparatus 1".

The apparatus 1 binds first primary elements 100, intermediate elements 101a, 101b and second primary elements 102 of absorbent sanitary articles together.

The primary elements 100 can be arranged adjacent to each other or spaced apart on a continuous web A1, can be made from the entire continuous web A1 or can be made from portions of the continuous web A1.

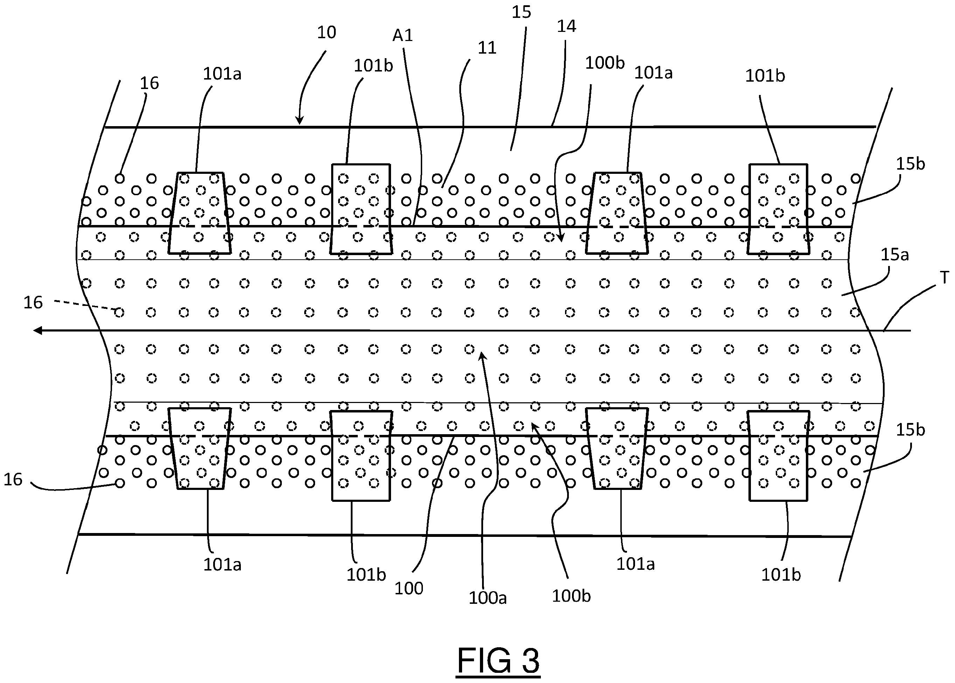

In the embodiment illustrated in the appended drawings, see in particular figure 3, the first primary elements 100 are made from the continuous web A1 and preferably form at least part of the topsheet of a nappy.

As illustrated in figure 3, the primary elements 100 comprise a central portion 100a and opposite side strips 100b.

The second primary elements 102 can be arranged adjacent to each other or spaced apart on a continuous web A2, can be made from the entire continuous web A2 or can be made from portions of the continuous web A2.

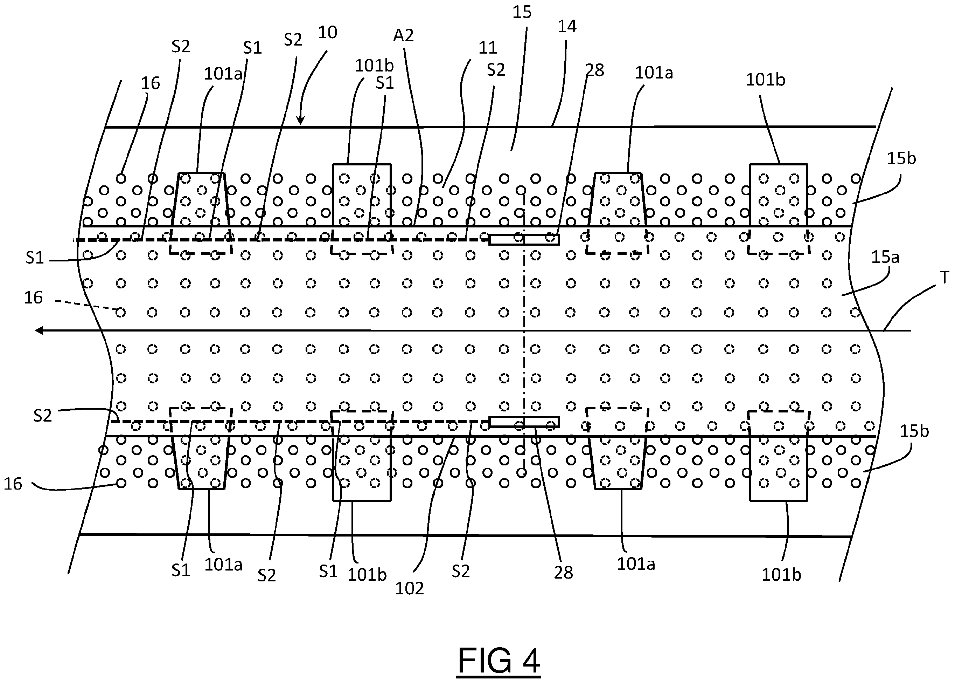

In the embodiment illustrated in the appended drawings, see in particular figure 4, the second primary elements 102 are made from the continuous web A2 and preferably form at least part of the backsheet of a nappy.

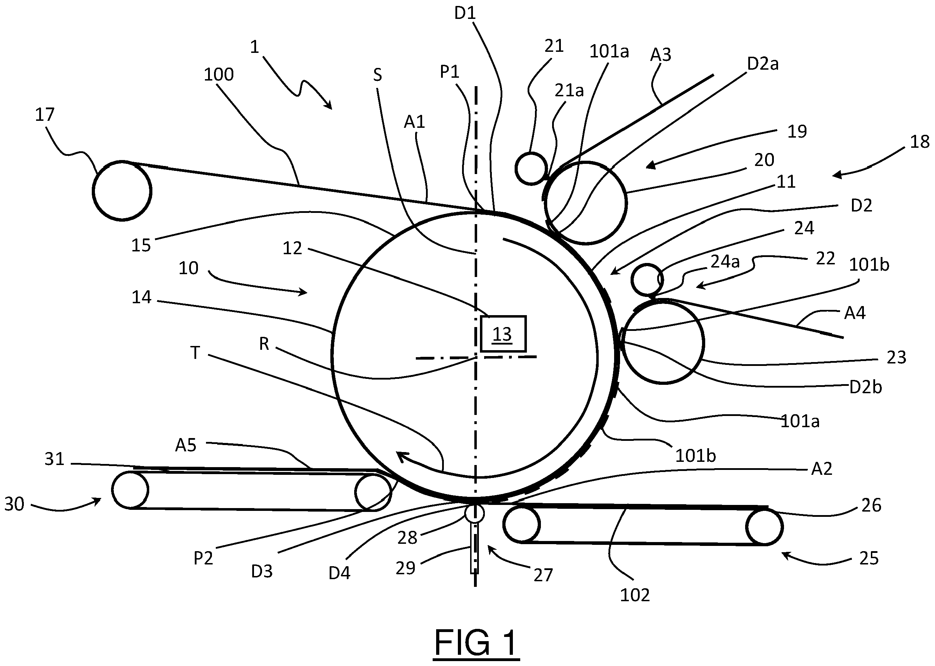

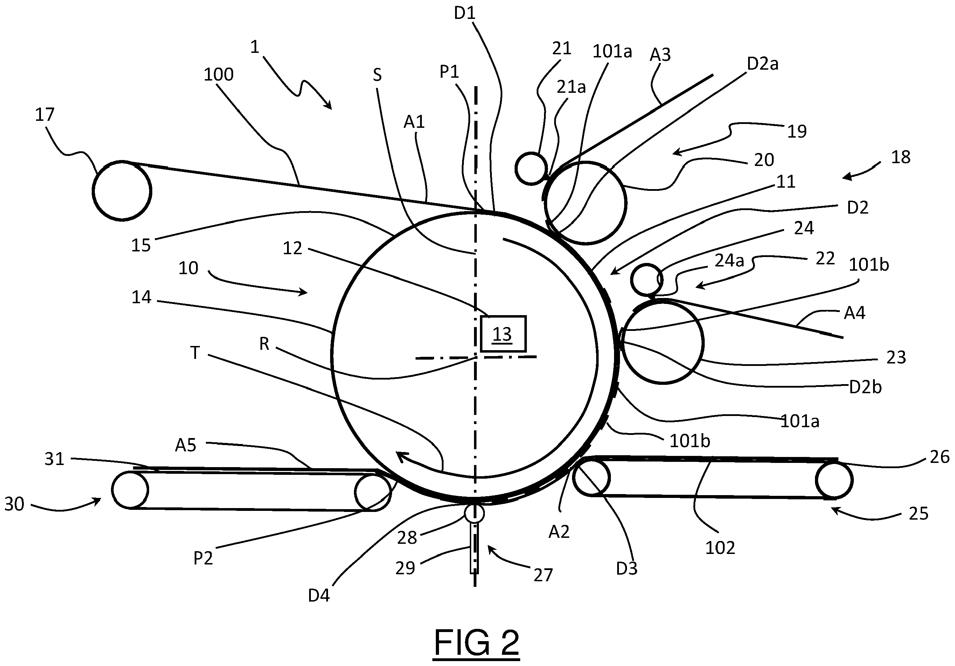

The intermediate elements 101a, 101b are in the form of discrete elements, in particular in the form of pairs of discrete elements parallel to each other (only one element of each pair of discrete elements is visible in figures 1 and 2). In the embodiment illustrated in the appended drawings, see in particular figures 3 and 4, the intermediate elements 101a, 101b respectively form the front and rear flaps of a nappy and are obtained from respective pairs of parallel continuous webs A3 and A4.

The apparatus 1 comprises a forming support 10 having a deposition surface 11.

The forming support 10 can be moved along a working direction T.

The forming support 10 comprises holding members 12 which act on the deposition surface 11 to hold the first primary elements 100, the intermediate elements 101a, 101b and the second primary elements 102 on the deposition surface.

The holding members 12 comprise a suction device 13 active on the deposition surface 11. The suction device 13 can be placed outside the forming support 10 and connected thereto, or it can be integrated into the forming support 10. In figures 1 and 2 the suction device has been schematically depicted inside the forming support 10. The suction device 13 can be any device capable of generating a vacuum, such as a suction pump.

The suction device 13 is configured to suck air through the deposition surface 11 so as to generate a vacuum (with respect to ambient pressure) near the deposition surface 11.

The suction exerted by the suction device 13 on the forming surface 11 is essentially perpendicular to the working direction T between a holding start position P1 and a holding end position P2. The holding start position P1 is placed along the working direction T upstream of the holding end position P2. The deposition surface 11 is movable along the working direction T, so that any portion of the deposition surface 11 passes from the holding start position P1 to the holding end position P2.

The suction device 13 selectively acts on the forming support 10 by sucking air through the deposition surface 11 along the entire portion of the deposition surface 11 which is located, from time to time, between the suction start position P1 and the suction end position P2 and by stopping the suction of air along the entire portion of the deposition surface 11 which is located, from time to time, between the suction end position P2 and the suction start position P1.

In the embodiment illustrated in the appended drawings, the forming support 10 comprises a drum 14 rotatable about a rotation axis R. The deposition surface 11 is defined by a cylindrical surface 15 of the drum 14. The working direction T is a circular, clockwise direction following the extension of the cylindrical surface 15 between the suction start position P1 and the suction end position P2.

On the cylindrical surface 15, a plurality of holding holes 16 is obtained in fluid communication with the suction device 13. The suction on the cylindrical surface 15 occurs by means of the suction holes 16, through which air is drawn from the environment immediately adjacent to the cylindrical surface 15. The suction action therefore has a direction perpendicular to the cylindrical surface 15 of the drum 14 and is directed radially towards the rotation axis R.

The holding holes 16 are obtained on the cylindrical surface 15 with different densities (i.e., number of holding holes 16 per surface unit of the cylindrical surface 15). On a central cylindrical portion 15a of the cylindrical surface 15, the holding holes 16 have a lower density than that at the side cylindrical portions 15b adjacent to the central cylindrical portion 15a and opposite each other. The suction action is greater at the side cylindrical portions 15b than at the central cylindrical portion 15a. The holding holes 16 are distributed along the entire central cylindrical portion 15a and along all the side cylindrical portions 15b of the cylindrical surface 15.

The rotation of the drum 14 feeds the holding holes 16 along the working direction T, such that any one of a plurality of holding holes 16, aligned or adjacent along a direction parallel to the rotation axis R, reaches the holding start position P1, proceeds to the holding end position P2, passes the holding end position P2, and again reaches the holding start position P1. When the holding holes 16 are located between the holding start position P1 and the holding end position P2, the suction device 13 is active on such holding holes 16. When the holding holes 16 are between the holding end position P2 and the holding start position P1, the suction device 13 is not active on such holding holes 16.

Therefore, the suction device 13 is active along the working direction T on the cylindrical surface 15 for a circumferential extension less than 360°.

A feeding member 17 is active on the continuous web A1 in order to feed it onto the forming support 10 at a deposition position D1. The feeding member 17 is configured to divert, unwind or convey the continuous web A1 towards the forming support 10 so that it reaches the forming support 10 at the deposition position D1.

In the embodiments illustrated in figures 1 and 2, the feeding member 17 is a transfer drum.

The deposition position D1 is placed at or downstream of the holding start position P1.

A feeding member 18 is configured to feed the intermediate elements 101a, 10ab onto the forming support 10. In particular, the feeding member 18 is configured to arrange sequences of pairs of intermediate elements 101a, 101b onto the deposition surface 11 at a deposition area D2.

The deposition area D2 is arranged downstream of the first deposition position D1 and downstream of the holding start position P1 and upstream of the holding end position P2.

The feeding member 18 comprises a first deposition member 19 configured to deposit onto the forming support 10 first pairs of intermediate elements 101a parallel to each other.

The first deposition member 19 is active on the forming support 10 at a position D2a of the deposition area D2.

The first deposition member 19 comprises a transfer drum 20 arranged in a position adjacent to the forming support 10. The transfer drum 20 is configured to receive a pair of continuous webs A3 parallel to each other. The first deposition member 19 further comprises a cutting member 21, comprising for example a pair of rollers equipped with a knife 21a, active on the transfer drum 20 and configured to cut the pair of continuous webs A3 into pairs of intermediate elements 101a.

The transfer drum 20 can be a so-called "cut and slip" drum, i.e., of the type configured to distance the pairs of intermediate elements 101a from each other before transferring them to the forming support 10.

The first deposition member 19 further comprises a unit for feeding (not illustrated) the pair of third continuous webs to the transfer drum 20. The feeding unit is configured to divert, unwind or convey the pair of continuous webs A3 to the transfer drum 20 upstream of the cutting member 21. The feeding unit can for example comprise a reel, a roller, a drum, a belt tensioner, a conveyor belt and/or at least one belt guide.

The feeding member 18 further comprises a second deposition member 22 configured to deposit onto the forming support 10 second pairs of intermediate elements 101b parallel to each other.

The second deposition member 22 is active on the forming support 10 at a second position D2b of the deposition area D2.

The second position D2b is placed downstream of the first position D2a.

The second deposition member 22 comprises a transfer drum 23 arranged in a position adjacent to the forming support 10. The transfer drum 23 is configured to receive a pair of continuous webs A4 parallel to each other. The second deposition member 22 further comprises a cutting member 24, comprising for example a pair of rollers equipped with a knife 24a, active on the transfer drum 23 and configured to cut the pair of continuous webs A4 into pairs of intermediate elements 101b.

The transfer drum 23 can be a so-called "cut and slip" drum, i.e., of the type configured to distance the pairs of intermediate elements 101b from each other before transferring them to the forming support 10.

The second deposition member 22 further comprises a unit for feeding (not illustrated) the pair of continuous webs A4 to the transfer drum 23. The feeding unit is configured to divert, unwind or convey the pair of continuous belts A4 to the transfer drum upstream of the cutting device 24. The feeding unit can for example comprise a reel, a roller, a drum, a belt tensioner, a conveyor belt and/or at least one belt guide.

A feeding member 25 is active on the continuous web A2 to feed the same onto the forming support 10 at a second deposition position D3. The feeding member 25 is configured to divert, unwind or convey the continuous web A3 towards the forming support 10 so that it reaches the forming support 10 at the deposition position D3.

The deposition position D3 is arranged downstream of the deposition position D1 and the deposition area D2. The deposition position D3 is arranged downstream of the holding start position P1 and upstream or at the holding end position P2.

In the embodiments illustrated in figures 1 and 2, the feeding member 25 is a conveyor belt 26.

The conveyor belt 26 can be equipped with a suction device to hold the continuous web A2 on the conveyor belt 26 before it is fed to the forming support 10. The conveyor belt 26 is configured to support the continuous web A2 in a horizontal position in order to improve the adherence of the continuous web A2 to the conveyor belt 26.

A bonding device 27 is arranged in the working direction T to bind the continuous web A1, the intermediate elements 101a, 101b and the continuous web A2 together.

In the embodiments illustrated herein, the bonding device 27 is an ultrasonic welding device.

As illustrated in figures 1, 2 and 4, the bonding device 27 comprises a pair of sonotrodes 28 and at least one pushing element 29 connected to the sonotrodes 28 to push them against the forming support 10.

The bonding device 27 is placed at a bonding position D4 along the working direction T.

The bonding position D4 is placed upstream of or at the holding end position P2.

According to a first embodiment, illustrated in figure 1, the bonding device 27 is placed at the deposition position D3. In other words, the web A2 is fed onto the forming support 10 at the bonding device 27.

According to a second embodiment, illustrated in figure 2, the bonding device 27 is arranged downstream of the deposition position D3. In other words, the web A2 is fed onto the forming support 10 upstream of the bonding device 27.

In both cases, the bonding device 27 is placed below the drum 14 and at a vertical symmetry plane S of the drum 14and therefore at 6 o'clock with reference to a clock face, as schematically illustrated in figures 1 and 2.

In order to bind the first primary elements 101, the intermediate elements 101a, 101b and the second primary elements 102 together, the continuous web A1 is fed to the forming support 10 at the deposition position D1. In this position, the suction device 13 is active on the forming support 10, which thus generates a vacuum on the deposition surface 11.

The continuous web A1 is sucked in and held onto the deposition surface 11. The suction force is such as to avoid a relative sliding between the continuous web A1 and the deposition surface 11.

As illustrated in figure 3, the continuous web A1 is fed onto the deposition surface 11 so that it is deposited onto the central cylindrical portion 15a of the cylindrical surface 15 of the drum 14.

The continuous web A1 is transported along the working direction T by the rotation of the drum 14 around the rotation axis R. During such a movement, the continuous web A1 is held onto the cylindrical surface by the suction action exerted through the holding holes 16.

In the preferred embodiment of the invention, the continuous web A1 is made of a fabric at least partially permeable to air, for example non-woven fabric having, at the opposite side portions defining the side strips 100b of the primary elements 100 a grammage for example of 15 gsm. Such a fabric defines at least part of the nappy topsheet.

The continuous web A1 is transported onto the forming support 10 towards the deposition area D2.

In the deposition area D2, the pairs of intermediate elements 101a, 101b are sequentially fed onto the forming support 10.

The intermediate elements 101a, 101b, before being deposited onto the deposition surface 11, are obtained from the continuous webs A3 and A4, respectively. The continuous web A3 is cut and divided into the intermediate elements 101a by the cutting elements 21, and the intermediate elements 101a thus obtained are transferred from the transfer drum 20 to the deposition surface 11 at the deposition position D2a. Similarly, the continuous web A4 is cut and divided into the intermediate elements 101b by the cutting elements 24 and the intermediate elements 101b thus obtained are transferred from the transfer drum 20 to the deposition surface 11 at the deposition position D2b.

The intermediate elements 101a, 101b comprise, in the preferred embodiment of the invention, first pairs of intermediate elements 101a defining, for example, front flaps of a nappy, and second pairs of intermediate elements 101b defining, for example, rear flaps of nappies.

The continuous web A3 is preferably made of a fabric which is at least partially permeable to air, e.g., a non-woven fabric having a grammage of 50 gsm, while the continuous web A4 is preferably made of a fabric with low permeability, i.e., having a grammage greater than 70 gsm and even up to 130 gsm. The continuous web A4 can also incorporate an elastic film which makes it waterproof. Such an elastic film is not present at the portions of continuous web A4 intended to be reciprocally bound to the continuous webs A1 and A2.

The intermediate elements 101a, 101b, once transferred to the deposition surface 11, are held thereon by the suction action exerted by the suction device 13.

As shown in figure 3, each pair of intermediate elements 101a, 10ab is deposited on the deposition surface 11 so that a portion of each intermediate element 101a, 10ab is placed on a respective side cylindrical portion 15b of the cylindrical surface 15 forming the deposition surface 11. Thus, a part of the intermediate elements 101a, 101b overlaps the side strips 100b of the first primary elements 100 obtained from the continuous web A1 and the remaining part of the intermediate elements 101a, 101b comes into direct contact with the forming support 10.

The holding of the intermediate elements 101a, 101b on the deposition surface 11 is implemented by the direct action of the holding holes 16 placed at the respective side cylindrical portion 15b of the cylindrical surface 15 and is assisted by the indirect action of the holding holes 16 placed at the central cylindrical portion 15a of the cylindrical surface 15. Such an indirect action is permitted by the fact that the first continuous web A1 is made of a material which is at least partially permeable to air.

As a result of the aforesaid holding action, the intermediate elements 101a, 101b are held onto the forming support 10 without any possibility of relative sliding between the intermediate elements 101a, 101b and the continuous web A1.

The intermediate elements 101a, 101b are fed and deposited onto the forming support 10 according to a predetermined deposition pattern. In such a predetermined pattern, the reciprocal position of the intermediate elements 101a, 101b is repeated substantially unchanged along the working direction T. An arrangement of intermediate elements 101a, 101b is shown in figure 3, in which pairs of intermediate elements 101a are equidistant from each other and pairs of intermediate elements 101b are equidistant from each other.

The intermediate elements 101a, 101b are transferred along the working direction T towards the deposition position D3 by the rotation of the drum 14.

The continuous web A2 is fed to the forming support 10 at the deposition position D3.

The continuous web A2 is overlapped on the continuous web A1 and partially overlaps the intermediate elements 101a, 101b. The continuous web A2 is placed onto the forming support 10 at the central cylindrical portion 15a of the cylindrical surface 15 of the drum 14.

Therefore, first simultaneous overlapping regions are determined, defined at opposite side portions of the continuous webs A1 and A2 in which opposite side portions of the continuous web A1 (in particular the side strips 100b of the first primary elements 100), portions of the intermediate elements 101a, 101b and opposite side portions of the continuous web A2 are reciprocally overlapped, and a second simultaneous overlapping region defined at the central portions of the continuous webs A1 and A2 in which the continuous web A2 is directly overlapped on the continuous web A1 without interposition of the intermediate elements 101a, 101b.

The action of the suction device 13 is indirect on the continuous web A2, i.e., the suction action acts on the continuous web A2 through the continuous web A1, which as already mentioned is at least partially permeable to air, and at the aforesaid first regions also through the intermediate elements 101a, 101b, which are also at least partially permeable to air. Although indirect, the suction force is such as to avoid a relative sliding between the continuous web A2, the intermediate elements 101a, 101b and the continuous web A1.

In the preferred embodiment of the invention, the continuous web A2 is made of a fabric impermeable to air, e.g., polyethylene (PE) film, with a grammage for example of 20 gsm, and at least partly defines the backsheet of nappies.

In the embodiment of figure 1, the deposition position D3 substantially coincides with the holding position D4 in which the bonding device 27 is active.

In the embodiment of figure 2, the deposition position D3 is placed upstream of the holding position D4. In this embodiment, the continuous web A1, the intermediate elements 101a, 101b and the continuous web A2 are integrally transferred onto each other, through the rotation of the drum 14, to the bonding device 27.

At this point the continuous web A1, the intermediate elements 101a 101b and the continuous web A2 are fed between the sonotrodes 28 and the forming support 10. During such feeding, the continuous web A1, the intermediate elements 101a, 101b and the continuous web A2 continue to be kept integral with each other and held onto the forming support 10 by the suction action exerted by the suction device 13.

The sonotrodes 28 make a pair of ultrasonic welds S1 at the opposite simultaneous overlapping regions of the continuous web A1, the intermediate elements 101a, 101b and the continuous web A2.

The continuous web A1, the intermediate elements 101a, 101b and the continuous web A2 are thus bound together with the intermediate elements 101a, 101b partially interposed between the continuous web A1 and the continuous web A2 and simultaneously bound to both the continuous web A1 and the continuous web A2.

The sonotrodes 28 make further ultrasonic welds S2 at the overlapping regions of the continuous web A1 and the continuous web A2 not affected by the intermediate elements 101.

As illustrated in figure 4, the welds S1 and the further welds S2 are made continuously, i.e., along parallel weld lines comprising both the simultaneous overlapping regions of the continuous web A1, the intermediate elements 101a, 101b and the continuous web A2, and the regions only overlapping the continuous web A1 and the continuous web S2.

The continuous web A1, the intermediate elements 101a, 101b and the continuous web A2, after having been bound together by the bonding device 27, form a continuous web assembly A5 from which a plurality of absorbent sanitary articles can be obtained which are substantially identical to each other.

In the embodiments of figures 1 and 2, the holding end position P2 is placed downstream of the bonding device 27.

In these embodiments, the continuous web assembly A5 is held onto the forming support 10 for a sufficient distance to allow the welds to consolidate.

The continuous web assembly A5 is subsequently picked up from the forming support 10, at the holding end position P2, and transferred to a pick-up device 30. This occurs, for example, through a suction action on the continuous web assembly A5 through a perforated surface 31 of the pick-up device 30 connected to a further suction device (not illustrated).

A knife (not illustrated) can be included on the pick-up device 30 for making transverse cuts or weakening lines on the continuous web assembly A5, so that the absorbent sanitary articles can subsequently be separated or made easily separable.

Obviously, a person skilled in the art, in order to satisfy specific and contingent needs, can make numerous modifications and variations to the invention described above while remaining within the scope of protection defined by the following claims.

## Claims

1. A method for binding together a plurality of elements of absorbent sanitary articles, comprising:
- feeding first primary elements (100) of absorbent sanitary articles onto a forming support (10), said first primary elements (100) being in the form of a first continuous web (A1);
- feeding a sequence of intermediate elements (101a, 101b) of absorbent sanitary articles onto the forming support (10) in an at least partial overlapping relation with respect to the first primary elements (100), said intermediate elements (101a, 101b) being in the form of discrete elements;
- holding said intermediate elements (101a, 101b) onto the forming support (10) by preventing a relative sliding between said intermediate elements (101a, 101b) and said first primary elements (100);
- feeding second primary elements (102) of absorbent sanitary articles onto the forming support (10) in an at least partial overlapping relation with respect to the first primary elements (100) and the intermediate elements (101a, 101b), said second primary elements (102) being in the form of a second continuous web (A2);
- binding together simultaneously the first primary elements (100), the intermediate elements (101a, 101b) and the second primary elements (102) along at least one mutual overlapping region thereof arranged onto the forming support (10).

2. The method according to claim 1, comprising, before or after feeding the first primary elements (100) onto the forming support (10), moving the forming support (10) along a working direction (T) and, after feeding the first primary elements (100) onto the forming support (10), holding said first primary elements (100) onto the forming support (10) by preventing a relative sliding between the first primary elements (100) and the forming support (10) during the movement of the forming support (10) along said working direction (T).

3. The method according to claim 2, wherein holding said first primary elements (100) onto the forming support (10) comprises exerting a suction action towards the forming support (10) on said first primary elements (100).

4. The method according to claim 2 or 3, comprising, after binding together the first primary elements (100), the intermediate elements (101a, 101b) and the second primary elements (102), temporarily holding the first primary elements (100), the intermediate elements (101a, 101b) and the second primary elements (102) bound together onto the forming support (10) before moving them away from the forming support (10).

5. The method according to any one of the previous claims, wherein:
- feeding the first primary elements (100) comprises depositing said first primary elements (100) onto the forming support (10) at a first deposition position (D1);
- feeding the sequence of intermediate elements (101a, 101b) comprises depositing said intermediate elements (101a, 101b) onto the forming support (10) at least one deposition area (D2) arranged downstream of said first deposition position (D1) with respect to said working direction (T);
- feeding the second primary elements (102) comprises depositing said second primary elements (102) onto the forming support (10) at a second deposition position arranged downstream of said deposition area (D2) with respect to said working direction (T);
wherein depositing said intermediate elements (101a, 101b) onto the forming support (10) comprises:
- depositing a first pair of intermediate elements (101a) at a first position (D2a) of said deposition area (D2);
- depositing a second pair of intermediate elements (101b) at a second position (D2b) of said deposition area (D2) arranged downstream of said first position (D2a) of said deposition area (D2).

6. The method according to any one of the previous claims, wherein feeding the second primary elements (102) comprises arranging the second primary elements (102) in a position corresponding to the one in which the first primary elements (100), the intermediate elements (101a, 101b) and the second primary elements (102) will be bound together.

7. The method according to any one of the previous claims, wherein the first primary elements (100) and the intermediate elements (101a, 101b) are at least partially permeable to air and the second primary elements (102) are substantially impermeable to air.

8. The method according to any one of the previous claims, wherein binding together the first primary elements (100), the intermediate elements (101a, 101b) and the second primary elements (102) comprises welding together the first primary elements (100), the intermediate elements (101a, 101b) and the second primary elements (102).

9. An apparatus (1) for binding together a plurality of elements of absorbent sanitary articles, comprising:
- a forming support (10) movable along a working direction (T);
- a first feeding member (17) configured to feed first primary elements (100) of absorbent sanitary articles onto the forming support (10) at a first deposition position (D1), said first primary elements (100) being in the form of a first continuous web (A1);
- at least one second feeding member (18a, 18b) configured to feed a sequence of intermediate elements (101a, 101b) of absorbent sanitary articles onto the forming support (10) at a deposition area (D2) arranged downstream of said first deposition position (D1) with respect to said working direction (T), said intermediate elements (101a, 101b) being in the form of discrete elements;
- a third feeding member (25) configured to feed second primary elements (102) of absorbent sanitary articles onto the forming support (10) at a second deposition position (D3) arranged downstream of said deposition area (D2) with respect to said working direction (T), said second primary elements (102) being in the form of a second continuous web (A2);
- a bonding device (27) operating onto the forming support (10) at the second deposition position (D3) or downstream of said second deposition position (D3) with respect to said working direction (T), said bonding device (27) being configured to bind the first primary elements (100), the sequence of intermediate elements (101a, 101b) and the second primary elements (102) together along at least one mutual overlapping region thereof defined onto the forming support (10).

10. The apparatus (1) according to claim 9, comprising holding members (12) configured to hold in position the first primary elements (100), the intermediate elements (101a, 101b) and the second primary elements (102) onto the forming support (10) during the movement of the forming support (10) along said working direction (T).

11. The apparatus (1) according to claim 10, wherein said holding members (12) comprise a suction device (13) operating on holding holes (16) formed on a deposition surface (11) of the forming support (10).

12. The apparatus (1) according to claim 11, wherein the forming support (10) comprises a drum (14) rotatable around a rotation axis (R) and wherein the suction device (13) operates in the working direction (T) on a cylindrical surface (15) of the drum (14) over a circumferential extension thereof lower than 360°.

13. The apparatus (1) according to any one of claims 9 to 12, wherein said at least one second feeding member (18) comprises:
- a first deposition member (19) configured to deposit a first pair of intermediate elements (101a) onto the forming support (10) at a first position (D2a) of said deposition area (D2);
- a second deposition member (21) configured to deposit a second pair of intermediate elements (101b) at a second position (D2b) of said deposition area (D2) arranged downstream of said first position (D2a) of said deposition area (D2) with respect to said working direction (T).

14. The apparatus (1) according to any one of claims 9 to 13, wherein the bonding device (27) is arranged below the forming support (10).

15. The apparatus (1) according to any one of claims 9 to 14, wherein the bonding device (27) comprises a welding device.
